# FASCICULE DE BREVET EUROPEEN

(11) **EP 0 437 599 B1**
(45) Date de publication et mention de la délivrance du brevet: **22.05.2002**
(21) Numéro de dépôt: 90913241.7
(22) Date de dépôt: 09.08.1990
(51) Int. Cl.: C12N 15/00, C12N 15/57

(54) **LIGNEES CELLULAIRES EXPRIMANT UN FACTEUR IX BIOLOGIQUEMENT ACTIF**
ZELLINIEN, DIE EINEN BIOLOGISCH AKTIVEN FAKTOR-IX EXPRIMIEREN
CELL LINEAGES EXPRESSING A BIOLOGICALLY ACTIVE FACTOR IX

(30) Priorité: 09.08.1989 FR 8910720
(43) Date de publication de la demande: 24.07.1991
(73) Titulaire: TRANSGENE S.A., 92400 Courbevoie (FR)
(72) Inventeur: JALLAT, Sophie, F-67000 Strasbourg (FR); MEULIEN, Pierre, F-75013 Paris (FR); PAVIRANI, Andréa, F-67000 Strasbourg (FR); PERRAUD, Frédéric, F-67000 Strasbourg (FR)
(74) Mandataire: Warcoin, Jacques
(86) Numéro de dépôt international: FR9000606
(87) Numéro de publication internationale: WO9102056

(56) Documents cités:
- EP-A- 0 167 420
- EP-A- 0 298 807
- EP-A- 0 307 248
- EP-A- 0 316 558
- GB-A- 2 223 755
- Nucleic Acids Research, Volume 15, no. 3, 1987, (Oxford, GB), K.H. CHOO et al.: "Expression of Active Human Blood Clotting Factor IX in Transgenic Mice: use of a cDNA with Complete mRNA Sequence", pages 871-884 voir l'article en entier (cite dans la demande)
- The EMBO Journal, Volume 3, No. 5, 1984, IRL Press Ltd., (Oxford, GB), D.S. ANSON et al.: "The Gene Structure of Human Anti-Haemophilic Factor IX" pages 1053-1060 voir l'article en entier
- Blood, Volume 72, No. 3, Septembre 1988, Grune & Stratton, Inc., (New York, US), P.H. REITSMA et al.: "The Putative Factor IX Gene Promoter in Hemophilia B Leyden", pages 1074-1076 voir Abrege; page 1074, colonne 2; page 1075, figure 1
- Embase Databank, Abrege No. 90288437, S. JALLET et al.: "Characterization of Recombinant Human Factor IX Expressed in Transgenic Mice and in Derived Trans-Immortalized Hepatic Cell lines", voir Abrege & Embo J., 1990, 9/10, 3295-3301
- DALEMANS ET AL. BIOLOGICALS vol. 18, 1990, pages 191 - 198

## Description

La présente invention concerne de nouvelles lignées cellulaires immortalisées obtenues à partir d'animaux transgéniques non humains qui expriment un facteur IX biologiquement actif, ainsi qu'un nouveau procédé de production du facteur IX à partir des lignées cellulaires selon l'invention.

Le facteur IX humain est une protéine de 415 acides aminés (forme mature), naturellement présente dans le sang, qui intervient dans la cascade des réactions aboutissant à la coagulation du sang. Le facteur IX est essentiellement synthétisé dans le foie, initiallement sous forme d'un précurseur qui est ensuite soumit à au moins quatre modifications post-traductionnelles: (i) une γ-carboxylation portant sur 12 résidus d'acides glutamique, (ii) une glycosylation, (iii) une β-hydroxylation du résidu d'acide aspartique en position 64 ainsi que (iv) l'abandon par clivage protéolytique du peptide signal suivi de celui du pro-peptide. Le facteur IX ainsi procéssé est sécrété dans le sang. Lorsqu'un événement de coagulation du sang a lieu, la forme circulante, à chaine unique, du facteur IX (forme zymogène) est clivée en présence du facteur XI activé pour donner la forme activée, à double chaine, du facteur IX (facteur IXa) qui intervient alors dans un complexe comprenant aussi du facteur VIIIa, des phospholipides provenant de la membrane des plaquettes sanguines et du calcium.

La déficience héréditaire en facteur IX humain est la cause d'une maladie grave, l'hémophilie de type B, qui affecte un sujet mâle sur trente mille. A l'heure actuelle, les patients sont traités par infusion de préparations concentrées de facteur IX dérivées du plasma humain. Les patients sont ainsi soumis à des risques d'infection non négligables en raison de l'éventuelle présence d'agents pathogènes non détectables dans le sang des donneurs. Afin de pallier à cet inconvénient majeur, des recherches ont été depuis longtemps entreprises pour produire le facteur IX par les techniques de l'ADN recombinant. Différents systèmes d'expression ont été proposés, notamment des systèmes mettant en jeu des cellules hôtes de mammifère. Néanmoins, on a constaté rapidement que le facteur IX produit de cette manière n'était pas correctement processé et que son activité élait largement infériéure à celle du facteur IX dérivé du plasma sanguin.

En parallèle, l'expression du facteur IX humain dans des animaux transgéniques a été aussi ohtenue. Choo et al., Nucl. Acids Res. (1987) 15: 871 rapporte l'expression de cette protéine dans des souris transgéniques dans lesquelles on avait au préalable introduit l'ADN complémentaire (cADN) codant pour le facteur IX placé sous le contrôle du promoteur inductible du gène de la métallothionéine la de mouton. A partir de ce promoteur, un niveau significatif de transcription n'est induit qu'en présence de zinc. Bien que le facteur IX ainsi produit soit biologiquement actif, l'expérience de Choo et al., bien que scientifiquement intéréssante, ne peut donc pas avoir d'application industrielle pour des raisons évidentes d'efficacité et d'éthique.

Par ailleurs, la demande de brevet européen n° 0 298 807 décrit un procédé de préparation de lignées cellulaires stables pour la production de protéines déterminées, notamment l'α-antitrypsine par préparation d'un vecteur comportant une séquence oncogène et une séquence d'ADN complémentaire de la protéine considérée.

Enfin, le document EP-A-316 558 décrit le clonage de l'ADNc du gène codant pour le facteur IX humain à des fins de diagnostic et de production de facteur IX par voie recombinante. A ce titre, les auteurs suggèrent différents systèmes d'expression à mettre en oeuvre dans les bactéries ou cellules mammifères.

Il a maintenant éte trouvé que des lignées cellulaires immortalisées dérivées du foie d'animaux transgéniques ayant intégré dans leur génome un ou plusieurs fragments d'ADN comprenant une séquence de type génomique ou minigène codant pour le facteur IX humain et un second fragment d'ADN exogène comprenant une région codant par un oncogène peuvent, lorsque mises en culture, produire un facteur IX dont l'activité est similaire à celle du facteur IX présent dans le plasma sanguin humain.

Par conséquent, l'invention propose une lignée cellulaire trans-immortalisée d'origine hépatique, capable d'exprimer un facteur IX d'origine humaine dont l'aclivité est similaire à celle du facteur IX présent dans le plasma humain, qui a intégré dans son génome un fragment d'ADN exogène comprenant une région de type génomique ou minigène codant pour le facteur IX humain, ladite séquence étant placée sous le contrôle d'une région promotrice spécifique du foie, et un second fragment d'ADN exogène comprenant une région codant pour un oncogène, ladite région codante étant placée sous. le contrôle d'une région promotrice appropriée.

Par "facteur IX d'origine humaine", on entend toute protéine présentant les deux caractéristiques suivantes :
- La protéine est sélectionnée parmi le facteur IX humain dont la séquence en acides aminés est donnée dans Anson et al., EMBO J. (1984) 3: 1053, et tous les analogues de celui-ci qui présentent des modifications dans la séquence en acides aminés. Ces modifications peuvent être des délétions, des insertions, ou des remplacements d'acides aminés par d'autres. Par "analogue du facteur IX humain", on entend également toute protéine ayant la même séquence en acides aminés que le facteur IX humain mais présentant des modifications post-traductionnelles différentes.
- La protéine a une activité coagulante telle que mise en évidence par le test de coagulation au kaolin (dosage un temps) décrit par Austen & Rhymes, (1975) A laboratory manual of blood coagulation, Blackwell Scientific Ed.; cette activité est, dans la suite du texte, appellée activité "facteur IX". A titre indicatif, une description détaillée d'un mode opératoire particulier de ce test est fournit ci-après.

100µl d'un plasma déficient en facteur IX (Stago) et 100µl d'une solution de céphaline-kaolin [25mg de kaolin (Merck; référence 1906); 100µl de céphaline (Stago) pour un volume final de 10ml dans du tampon Owren & Koller (5,5g/l d'acide 5,5 diethylbarbiturique, 3,69g/l d'acétate de sodium, NaCl 0,63%, pH7,3) sont ajoutés à 100µl d'un aliquot d'un échantillon devant être testé. Si besoin est, cet échantillon peut être au préalable dilué dans du tampon imidazol (1,75 g/l; pH 7,4). On incube cette préparation 180 secondes à 37°C, puis on ajoute 100µl de CaCl₂ 0,025M. Le temps de latence avant formation d'un caillot (temps de coagulation) est mesuré de manière automatique (Behring Fibrintimer 10). L'activité "facteur IX" est établie, par la suite, en fonction d'une gamme d'étalonnage préparée comme suit: Un plasma contrôle contenant le facteur IX (plasma contrôle N FVIII-FIX de Stago, dont l'activité est donnée par le fournisseur; 1U = 5µg de facteur IX) est dilué au 1/10, 1/20, 1/40, 1/80 et 1/160 dans du tampon imidazol. Le temps de coagulation est mesuré pour chacune des dilutions et la courbe d'étalonnage est établie (Unité "facteur IX" en fonction du temps de coagulation sur une échelle logarythmique). Lorsque le mode opératoire ci-dessus décrit est mis en oeuvre, on considère qu'un échantillon testé n'a pas d'activité "facteur IX" si l'activité mesurée est inférieure à 250 ng/ml.

Lorsque ce test est réalisé avec le plasma d'un animal transgénique exprimant le facteur IX humain, il convient d'utiliser une double série d'échantillons; la première série comprenant du plasma tel que prélevé, et la deuxième série comprenant du plasma traité avec des anticorps anti-facteur IX humain neutralisants, dans la mesure où ces anticorps ne présente pas de réaction croisée avec le facteur IX des animaux en question. Ceci permet, en évaluant la différence des résultats obtenus en parallèle de révéler l'activité facteur IX due spécifiquement au facteur IX humain. Enfin, si besoin est, le plasma des animaux transgéniques est dilué de façon plus importante avec du tampon imidazol.

Par "facteur IX d'origine humaine ayant une activité similaire au facteur IX présent dans le plasma humain", on entend un facteur IX d'origine humaine qui est représenté par un échantillon donné dont l'activité facteur IX mesurée en µg/ml, en mettant en oeuvre le test de Austen & Rhymes précédemment décrit, équivaut à au moins 75%, de préférence à au moins 80%, ou mieux encore, à au moins 90% de la quantité totale de facteur IX dans l'échantillon, mesurée en µg/ml par test ELISA. Un kit ELISA spécifique pour le facteur IX est commercialement disponible chez Diagnostica Stago, Asnières, France. Considérant la définition du présent paragraphe, le facteur IX présent dans le plasma humain a une activité facteur IX d'environ 100%. D'une manière générale, un facteur IX répondant au critère d'activité énoncé ci-dessus, est désigné sous le terme "facteur IX biologiquement actif".

Par "région promotrice spécifique du foie", on entend un promoteur de mammifère ou partie de celui-ci, capable d'initier la transcription d'une séquence codante dans un nombre restreint de types de cellule de mammifère (pas dans tous les types), en particulier dans une cellule hépatique ou d'origine hépatique. Dans le cadre de la présente invention un promoteur spécifique du foie est un promoteur capable d'initier la transcription d'une séquence codante essentiellement dans une cellule hépatique ou d'origine hépatique; par exemple, le promoteur du gène de l'α-antitrypsine ou du facteur IX et les parties de ceux-ci suffisantes pour induire l'expression de ladite région codant. Aux fins de la présente invention, ce dernier promoteur est tout particulièrement préféré, bien que jusqu'à présent on ait supposé que l'expression contrôlée par le promoteur du gène du facteur IX soit plus faible que l'expression contrôlée par le promoteur du gène de l'α-antitrypsine

D'une manière tout à fait générale, une lignée cellulaire selon l'invention peut être obtenue par un procédé de trans-immortalisation in vivo. Elle peut donc être sélectionnée à partir d'une culture de cellules tumorales hépatiques prélevées sur un animal transgénique ou sur un animal dans lequel une tumeur hépatique issue d'un animal transgénique a été propagée.

Dans le cadre de la présente invention, on entend par "animal transgénique", un animal trangénique non humain qui possède, intégré dans son génome, un fragment d'ADN exogène comprenant une région codant pour un facteur IX d'origine humaine ou une région codant pour un oncogène et qui est capable d'exprimer les protéines correspondantes. Cet animal transgénique peut être un transgénique complet (toutes ses cellules possède le fragment d'ADN exogène) ou bien un mosaïque (le fragment d'ADN exogène se retrouve dans un certain pourcentage de cellules, mais pas dans toutes). Cet animal transgénique est de préférence un mammifère non-humain, plus particulièrement un murin, de manière tout à fait préférée une souris.

En tenant compte de ce qui précède, un animal transgénique à partir duquel une lignée cellulaire peut être dérivée doit être doublement transgénique: pour le facteur IX et pour un oncogène.

Selon un premier mode de réalisation, un tel animal est obtenu par développement d'un oeuf dans lequel on a injecté, à la fois, (i) un premier fragment d'ADN exogène comprenant une région codant pour le facteur IX et placée sous le contrôle d'un région promotrice spécifique du foie et (ii) un deuxième fragment d'ADN comprenant une région codant pour un oncogène (séquence oncogène) placée sous le contrôle d'un région promotrice appropriée, par exemple, un promoteur spécifique du foie. Le premier et le deuxième fragments d'ADN peuvent être injectés en tant que deux molécules d'ADN distinctes ou bien en tant qu'une entité d'ADN unique.

Selon un deuxième mode de réalisation alternatif, un animal transgénique pour le facteur IX, capable de développer des tumeurs hépatiques à partir desquelles on peut établir des lignées cellulaires selon l'invention, peut être obtenu comme suit: On injecte dans des oeufs un fragment d'ADN comprenant une région oncogène, afin d'obtenir un premier animal transgénique pour un oncogène. En parallèle, on injecte un fragment d'ADN comprenant une région codant pour le facteur IX afin d'obtenir un deuxième animal transgénique. Ensuite on croise le premier et le deuxième animal transgénique afin d'obtenir un descendant doublement transgénique pour l'oncogène et le facteur IX.

Par conséquent, l'invention concerne également:
un animal transgénique dont le plasma sanguin contient un facteur IX d'origine humaine dont l'activité est similaire à celle du facteur IX présent dans le plasma sanguin humain, qui a intégré dans son génome un premier fragment d'ADN exogène comprenant une région de type génomique ou minigène codant pour un facteur IX d'origine humaine; la dite région étant placée sous le contrôle d'une région promotrice spécifique du foie; et
qui est en plus capable de développer une tumeur hépatique, puisqu'il a intégré dans son génome un second fragment d'ADN exogène comprenant une région codant pour un oncogène placée sous le contrôle d'une région promotrice appropriée, par exemple une région promotrice eucaryote ou d'origine virale. De préférence c'est une région promotrice spécifique du foie.

D'une manière générale, le second fragment d'ADN comprend une région codant pour un oncogène capable d'induire une tumeur hépatique chez l'animal que l'on considère. Par exemple, cela peut être l'antigène T du virus du SV40 ou l'antigène codé par le gène c-myc de souris.

Aux fins de la présente invention, un fragment d'ADN comprenant une région codant pour le facteur IX humain peut être construit sous forme de bloc d'expression, plus précisément en plaçant la dite région sous le contrôle d'une région promotrice en 5' de la région codante qui peut être un promoteur spécifique du foie ou au moins une partie de celui-ci suffisante pour permettre l'expression du facteur IX dans une cellule hépatique. Outre une région promotrice et une région codante, le bloc d'expression peut aussi comprendre des eléments de fin de terminaison de transcription.

La séquence de la région codant pour le facteur IX est:
- de type génomique; par exemple, dans le cas du facteur IX humain, il s'agit, en particulier, de la séquence décrite dans Anson et al. (supra), qui comprend 8 exons (exons A à H) et 7 introns (introns 1 à 7) ou d'une séquence correspondant à la séquence ci-dessus en référence dans laquelle la taille d'au moins un intron a été réduite en introduisant une délétion.
- de type "mixte", (appellée aussi minigène); par. exemple, dans le cas du facteur IX humain, il s'agit de la séquence décrite dans Anson et al. (supra), dans laquelle au moins un des 7 introns a été supprimé et dans laquelle au moins un'des 7 introns a été conservé. De préférence, seul le premier intron ou partie du premier intron est conservé.

Selon un mode préféré de l'invention, on choisit de mettre en oeuvre une séquence de type génomique.

Le fragment d'ADN exogène peut en outre comprendre une région amplificatrice telle que une région codant pour l'adénosine déaminase (ADA) qui confère une résistance progréssive à certains antibiotiques. La région amplificatrice et la région codant pour le facteur IX doivent être placées sous le contrôle d'une région promotrice commune. Par sélection basée sur la resistance à de fortes doses d'antibiotique, on pourrait ainsi obtenir une lignée cellulaire hyperproductrice de facteur IX.

Ce fragment d'ADN exogène est initialement construit en tant que fragment inséré dans un vecteur d'expression, par exemple un plasmide. Néanmoins, pour usage dans la présente invention, il doit être mis sous forme linéaire et dissocié des autres éléments plasmidiques.

Par ailleurs, l'invention propose de même un procédé de préparation d'une lignée cellulaire selon l'invention dans lequel
- on prélève le foie tumoral (ou en cours de tumorigénèse) d'un animal transgénique selon l'invention ou le foie tumoral d'un animal dans lequel des cellules hépatiques tumorales dérivées d'un animal transgénique selon l'invention on été propagées et,
- on sélectionne, par mise en culture des cellules du dit foie, la dite lignée cellulaire.

Enfin, l'invention se rapporte également à un procédé de préparation d'un facteur IX d'origine humaine ayant une activité similaire à celle du facteur IX présent dans le plasma humain, dans lequel on cultive une lignée cellulaire selon l'invention et on récolte, par la suite, le dit facteur IX dans le milieu de culture.

L'invention est illustrée ci-après et se réfère aux figures suivantes:

La Figure 1 représente schématiquement la structure du gène codant pour le facteur IX humain; sont indiqués: la position des exons (de A à H), la localisation des séquences reconnues par les oligonucléotides utilisés pour cribler la banque génomique (de [1] à [6]), et les morceaux d'ADN clonés dans trois vecteurs lambda, λTG1951, λTG1958 et λTG1952.

La Figure 2 représente schématiquement la structure du plasmide pTG3960 en précisant de quels plasmides proviennent ses fragments et à partir de quel vecteur lambda ces derniers ont été construits. Les exons du facteur IX sont signalés par les lettres de A à H.

La Figure 3 représente schématiquement la structure du plasmide pTG3954 en précisant de quels vecteurs proviennent ses différents fragments. Les exons du facteur IX sont signalés par les lettres de A à H.

La Figure 4 représente schématiquement la structure du plasmide pTG3962 en précisant de quels plasmides proviennent ses différents fragments. Les exons du facteur IX sont signalés par les lettres de A à H (rectangles clairs), le premier exon non codant du gène de l'α-antitrypsine par le chiffre 1 et la partie de l'exon c-myc par le chiffre 2 (rectangles noirs).

La Figure 5 présente l'analyse par électrophorèse des ARN préparés à partir des différents tissus d'une lignée de souris transgéniques exprimant le facteur IX humain ("C", pour souris contrôle et "T", pour test).

La Figure 6 présente l'analyse des ARN messagers issus du foie de souris transgéniques exprimant le facteur IX humain; (1) et (2) souris de la lignée TMTG3960-1, "C" souris contrôle; (3) souris de la lignée TMTG3962-18; (4) souris de la lignée TMT03954-9; et (5) et (6) souris de la lignée TMTG3954-58.

La Figure 7 représente l'analyse par Western blot du facteur IX recombinant humain isolé du plasma des souris transgéniques; (1) et (2) souris de la lignée TMTG3954-58; "C" souris contrôle, (3), (4), (5) et (6) souris de la lignée TMTG3960-1, "S" facteur IX Stago et (7) et (8) souris de la lignée TMTG3954-9.

Les Figures 8a et 8b représentent shémaliquement de la construction du plasmide pTG4912.

### EXEMPLE 1: Préparation de vecteurs portant un fragment d'ADN comprenant une région codant pour le facteur IX humain

### A. Clonage d'une région codant pour le facteur IX humain

Une banque d'ADN génomique provenant d'une lignéc de cellules lymphoblastoïdes humaines GM1202A (4XY) est réalisée dans le bactériophage λEMBL3 [Pavirani A et al., Bio\Technology (1987) 5: 389], puis est criblée à l'aide des sondes d'oligonucléotides synthétiques (21, 22 et 25 mer) suivantes:

5' AAGTACAAGCTGAGCTGTATT 3' [1]
5' TGCCATGATCATGTTCACGCG 3' [2]
5' CTCAGTACAGGAGCAAACCACCTTG 3' [3]
5' TAGAATTTACATAGTCCACATCAGG 3' [4]
5' GTCTCTACTAGCTGTAGATTGC 3' [5]
5' GATTATAGGCGTGAGCCACTG 3' [6]

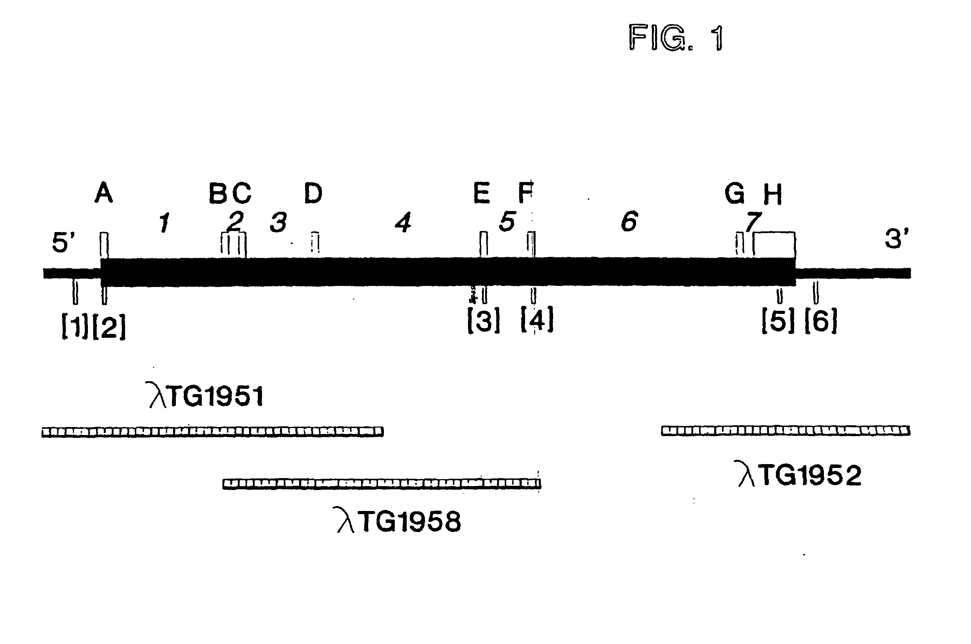

Celles-ci correspondent à différentes régions du gène du facteur IX, tel qu'indiqué à la Figure 1.

Trois clones λ indépendants sont identifiés à l'aide des sondes, par Southern blot: λTG1951, λTG1958 et λTG1952 (Figure 1). L'insert de λTG1951 comprend les 5 kb de la région promotrice et la région couvrant les quatre premiers exons du gène. L'insert de λTG1952 comprend 2,3 kb de l'intron 6, l'intron 7, les exons G et H et les 10 kb de la séquence adjacente en aval du signal de polyadénylation. L'insert de λTG1958 s'etend de l'exon B jusqu'à 0,47 kb en aval de l'exon F (il recouvre l'insert de λTG1951 sur 7 kb). En conclusion, les trois inserts ci-dessus décrits couvrent, à eux trois, une grande partie du gène, en particulier, les huit exons et les régions adjacentes 5' (5 kb) et 3' (10 kb); seule une région de l'intron 6 (6,7 kb) ne s'y retrouve pas.

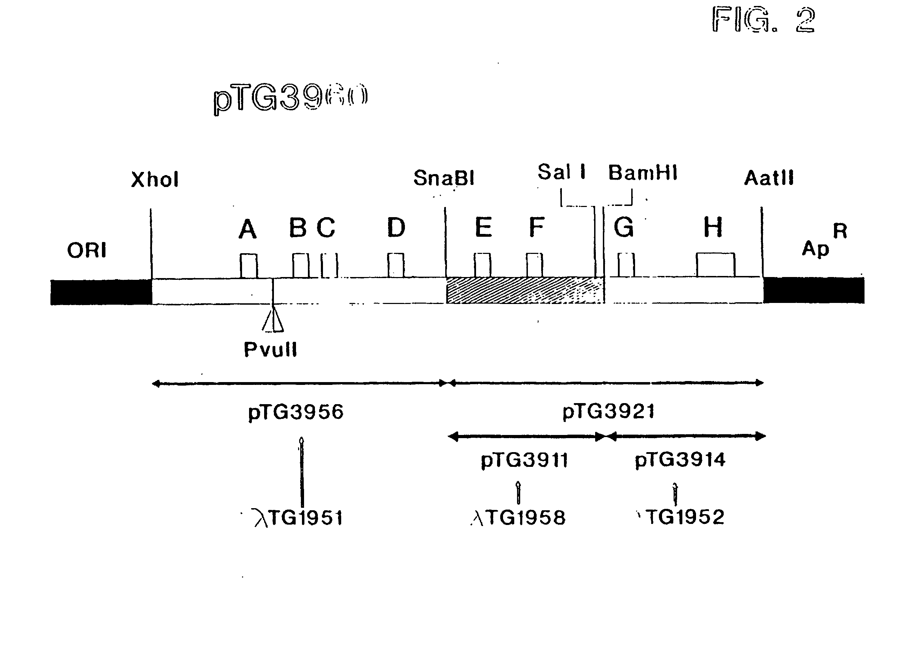

### B. Construction d'un plasmide comportant une séquence d'ADN de type génomique codant pour le facteur IX humain, placée sous le contrôle du promoteur du gène du facteur IX humain (pTG3960, Figure 2)

Le fragment SalI de 15 kb du clone λTG1958 est sous-cloné dans le site de restriction Sali du vecteur ppolyIII-I* décrit dans Lathe et al., Gene (1987) 57: 193 pour donner le plasmide pTG3911.

Un linker synthétique porteur de sites de restriction uniques (SfiI, EcoRI, KpnI, BamHI, XhoI et NotI) est introduit entre les deux sites EcoRI du cosmide pCV001 [Choo et al., Gene (1986) 46: 277] qui sont détruits lors de l'insertion, pour générer le plasmide pTG3909. Les fragments BamHI-EcoRI (4,3 kb) et EcoRI-EcoRI (1,8 kb) isolés du clone λTG1952 sont clonés entre les sites BamHI-EcoRI de pTG3909 pour donner pTG3914. L'orientation du fragment EcoRI-EcoRI est vérifiée par digestion enzymatique. Le fragment de 6,2 kb (les deux fragments précédents plus un fragment de vecteur, EcoRI-AatII) portant la partie 3' du gène du facteur IX (correspondant aux exons G et H) est resorti du cosmide par digestion avec BamHI et AatII. Puis ce fragment est cloné entre les mêmes sites de pTG3911 pour donner le plasmide pTG3921.

L'insert SalI-SalI de λTG1951 est sous-cloné dans le site Sali du plasmide pAT153 [Twigg A.J. et Sherrat D., Nature (1980) 283: 216], pour donner le plasmide pTG3927. La partie centrale de l'intron 1 est éliminée par digestion PvuII suivie d'une ligation, pour donner le plasmide pTG3952. Ce plasmide n'a plus les deux fragments PvuII (3,65 kb et 1,13 kb) internes à l'intron 1 mais retient le fragment PvuII (4,38 kb) porteur des exons B et C. L'insert SalI-SalI de pTG3952 est repassé dans le vecteur ppolyIII-I* pour donner le plasmide pTG3956 qui dispose de sites uniques en 5'. Le fragment XhoI-SnaBI de pTG3956 est introduit entre les sites XhoI-SnaBI de pTG3921 pour donner le plasmide pTG3960 (Figure 2) qui comporte :
- 5 kb de la séquence promotrice du gène du facteur IX,
- 22 kb du fragment codant pour le facteur IX (l'intron 1 est tronqué de ces deux fragments PvuII et l'intron 6 de 7,1 kb du fait de la délétion supplémentaire d'un fragment de 0,46 kb due à l'utilisation du site BamHI le plus en aval de l'intron,
- quelques centaines de paires de bases de séquences génomiques 3' en aval du signal de polyadénylation.

Toutes les constructions sont réalisées par passage dans la souche E. coli 5K, à l'exception de la dernière étape de clonage qui utilise la souche E. coli BJ.

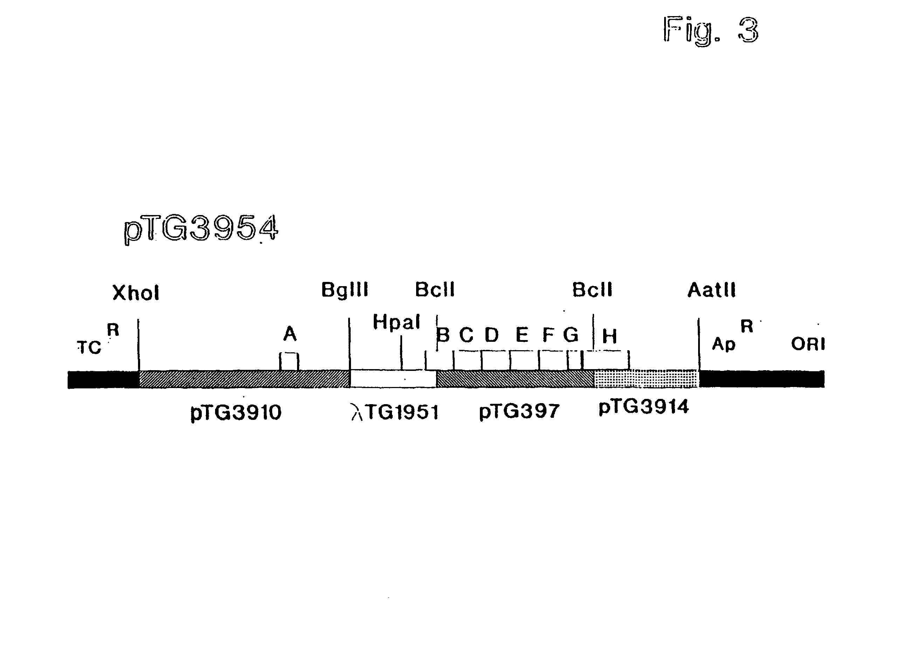

### C. Construction d'un plasmide comportant une séquence de type "mixte" codant pour le facteur IX humain, placée sous le contrôle du promoteur du gène du facteur IX humain (pTG3954, Figure 3)

Le fragment de 6,2 kb comportant l'extrémité 3' du gène du facteur IX est resorti du cosmide pTG3914 par digestion avec ClaI et AatII. Puis il est cloné entre les sites ClaI et AatII du vecteur pAT153, pour générer le plasmide pTG3924. Cette construction est passée dans la souche E. coli GM33 (dam⁻). Les séquences génomiques comprises entre les sites BclI et BglII sont éliminées et remplacées par un fragment BclI-BglII (2,34 kb) provenant du phage λTG1951 et comportant l'extrémité 5' de l'exon B et la partie 3' de l'intron 1, pour donner le plasmide pTG3926. Ce clonage est effectué dans la souche E. coli 5K. Il subsiste 0,6 kb de séquences génomiques issues de l'intron 6 entre les sites BamHI et BglII qui sont éliminées lors de la dernière étape de clonage. Le plasmide pTG3926 est transféré dans la souche E. coli GM33 (dam⁻) afin de pouvoir reconstituer la partie de l'ADN complémentaire entre les exons B et H en intégrant le fragment BclI (1.4 kb) isolé du plasmide pTG397 décrit dans la demande européenne de brevet EP-A- 162 782 (ce plasmide comporte l'ADN complémentaire du facteur IX humain) pour donner le plasmide pTG3951.

La partie promotrice, l'exon A et le reste de l'intron 1 sont isolés du plasmide pTG3910 (pTG3910 provient du clonage du fragment SalI de λTG1951 dans le vecteur ppolyIII-I*) sous forme d'un fragment XhoI-BglII. Ce fragment est intégré entre ces mêmes sites dans pTG3951 pour donner le plasmide pTG3954 (Figure 3) qui comporte donc un minigène du facteur IX comprenant la partie de 5 kb du promoteur, l'exon A, le premier intron complet, les exons de B à H sous forme d'un ADN complémentaire et quelques centaines de paires de bases de la partie 3' adjacente en aval du signal de polyadénylation.

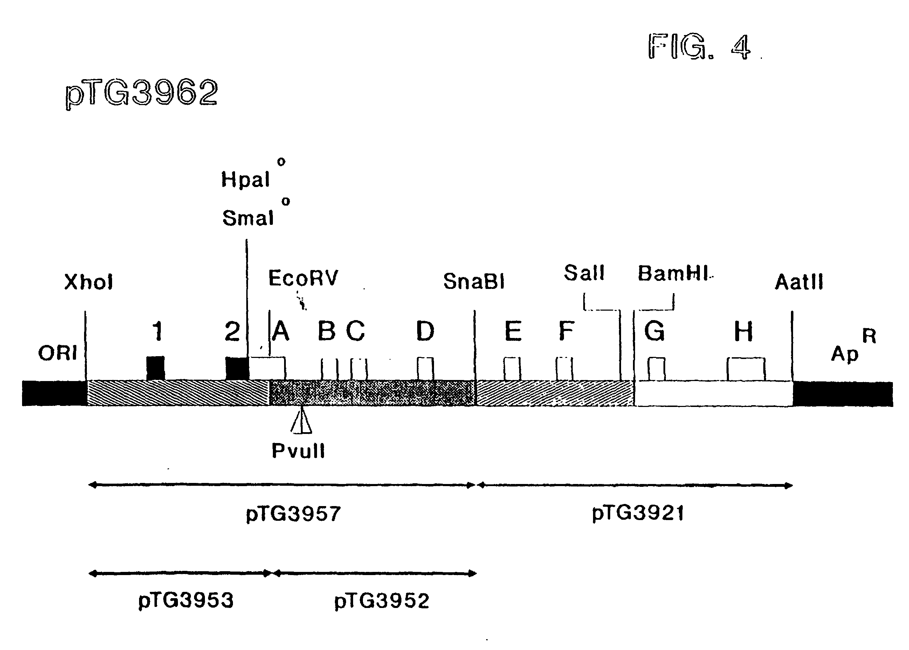

### D. Construction d'un plasmide comportant une séquence d'ADN de type génomique codant pour le facteur IX humain, placée sous le contrôle du promoteur du gène de l'α-antitrypsine (pTG3962, Figure 4)

Un fragment KpnI-KpnI comportant la région promotrice, le premier exon non codant et la partie 5' de l'intron 1 du gène de l'α-antitrypsine dont la séquence est donnée par Long G.L. et al., Biochemistry (1984) 23: 4828, est inséré dans le vecteur pUC18 [Yanisch-Perron C. et al., Gene (1985) 33: 103]. En particulier, la partie 5' de l'intron 1 comporte un site donneur d'épissage.

Puis un intron fonctionnel hybride est créé en introduisant, dans le site Xbal du polylinker de pUC18, un adaptateur dont la séquence est basée sur celle de l'extrémité 3' du premier intron du gène de souris c-myc. Ce fragment synthétique a pour séquence :

XbaI                    (*)                HpaI   XbaI
5'TCTAGACTTGCTTCCCTTGCTGTGCCCCCTCCAGCAGACAGCCACGACGGTTAACTCTAGA
  └ extrémité 3' du premier intron ┘└ 2ième exon ┘
          du gène c-myc                c-myc

Ce fragment synthétique comporte, de 5' vers 3', un site XbaI, un site accepteur d'épissage (*) permettant l'epissage correct du transcript (la coupure se faisant entre G et C), un site de clonage aux extrêmités franches (HpaI) pour permettre la fusion avec le fragment d'ADN codant pour le facteur IX au niveau des régions exoniques, et enfin un site XbaI. L'orientation de ce fragment est vérifiée par séquençage. En fin de construction on obtient le plasmide pTG3925.

En parallèle, un fragment HindIII (4 kb) de λTG1951 portant la partie 5' de la région promotrice du gène du facteur IX, l'exon A, et environ 600 pb de l'intron 1 est sous-cloné dans le vecteur M13TG130 [Kieny M.P. et al., Gene (1983) 26: 91]. Puis un site SmaI (extrémités franches) est créé par mutagénèse dirigée en utilisant l'oligonucléotide 5'GCATAACCCGGGCTAGCAG 3'. Ce site est créé, deux nucléotides en amont de l'ATG initiateur de la transcription. L'insert HindIII ainsi modifié est réintégré dans le vecteur ppolyIII-I* pour donner le plasmide pTG3913.

La partie promotrice et le début de la région codante du gène de l'α-antitrypsine munie du site accepteur d'épissage sont isolées du vecteur pTG3925 sous forme d'un fragment EcoRI-HpaI. Ce fragment est intégré entre les sites EcoRI et SmaI du plasmide pTG3913 pour générer le plasmide pTG3953. Cette insertion détruit les sites de clonage HpaI et SmaI et se fait dans une zone exonique. L'ATG initiateur est fourni par la région codant pour le facteur IX (exon A). Le séquençage de la zone de fusion (entre la séquence synthétique correspondant à c-myc murin et l'exon A) indique qu'un nucléotide est perdu lors du clonage:

HpaI°SmaI°
...CAGACAGCCACGACGGTTGGTTATG...
c-myc ┘└ facteur IX

La partie 5' de la région codant pour le facteur IX, comportant l'intron 1 tronqué du gène du facteur IX, est reconstituée afin d'appliquer la même stratégie que précédemment pour l'assemblage de la région codante complète. A cet effet, le fragment EcoRV-SalI isolé du plasmide pTG3952 est cloné entre les mêmes sites de pTG3953, pour donner le plasmide pTG3957.

Enfin, le fragment XhoI-SnaBI (9,9 kb) de pTG3957 est transféré entre les mêmes sites de pTG3921, pour donner le plasmide pTG3962 (Figure 4). Cette dernière étape de clonage est réalisée par passage dans la souche E. coli BJ alors que les précédents sont réalisés par passage dans la souche E. coli 5K.

Dans pTG3962 (Figure 4) le premier exon non codant du gène de l'α-antitrypsine est conservé.

### EXEMPLE 2: Souris simple-transgéniques exprimant le facteur IX humain

### A. Obtention des souris transgéniques

pTG3954 est digéré par Sali et SfiI tandis que pTG3960 et pTG3962 sont digérés par SfiI uniquement, de manière à obtenir dans chaque cas, un fragment d'ADN comprenant un bloc d'expression du facteur IX dissocié des fragments d'ADN plasmidique derivés de pBR322. Chaque fragment ainsi libéré, est purifié sur gradient de sucrose et injecté dans le pronucléus mâle d'un oeuf fertilisé de souris hybrides de première génération issues du croisement d'une souris femelle C57/B16 avec un mâle SJL selon la technique connue de l'homme du métier. Chaque oeuf est ensuite réimplanté dans l'utérus d'une souris pour que la gestation soit poursuivie.

L'ADN des tissus de la queue des nouveau-nés est isolé de manière à vérifier l'intégration du fragment d'ADN exogène dans le génome de l'animal par analyse Dot-blot en utilisant une sonde appropriée (fragment BamHI-BamHI du plasmide pTG397, décrit dans la demande européenne de brevet EP-A-162 782) marquée à l'isotope ³²P. Du sang est prélevé sur les souris positives au test d'hybridation et le niveau d'expression du facteur IX est déterminé dans le sérum par la méthode ELISA en utilisant la trousse de dosage Diagnostica Stago (Asserachrom IX:Ag; Réf. 0410). Le test est spécifique du facteur IX humain et montre peu ou pas de réaction croisée avec le facteur IX de souris. Les résultats sont présentés ci-dessous.

**Tableau 1**

| Plasmide | n° de la fondatrice | sexe | expression du facteur IX (µg/ml) |
|---|---|---|---|
| pTG3960 | 1 | M | 6,97 |
| pTG3960 | 83 | F | 3,47 |
| pTG3962 | 18 | M | 1,82 |
| pTG3954 | 9 | M | 36,4 |
| pTG3954 | 58 | M | 8,02 |

Le tableau 1 montre que l'expression la plus élevée est obtenue avec les fragments d'ADN issus de pTG3954 et pTG3960 qui comportent le promoteur du gène du facteur IX humain. De plus, pour obtenir un bon niveau d'expression, il n'est pas nécessaire d'avoir la totalité des introns (pTG3954).

### B. Analyse de l'ARNm correspondant au facteur IX humain, issu des souris transgéniques

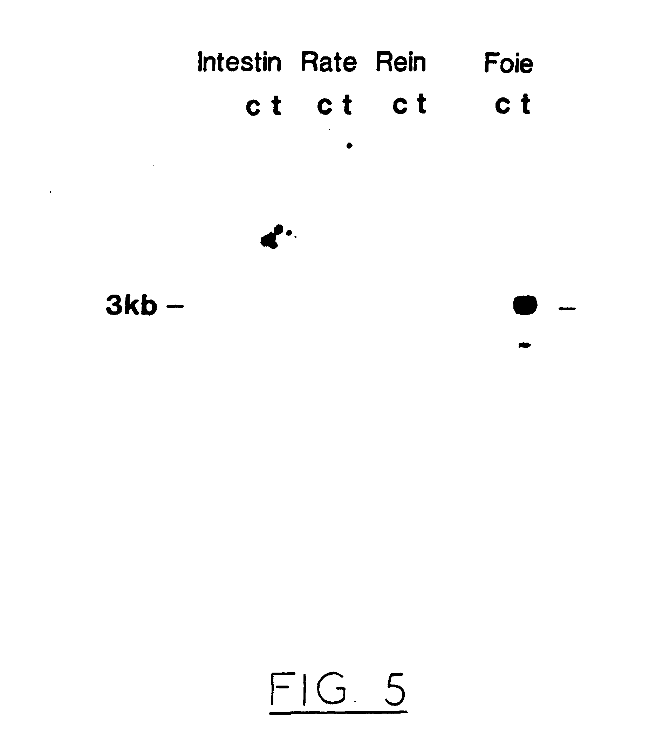

L'ARN total de plusieurs tissus (rein, rate, intestin et foie) est préparé à partir de souris transgéniques de la lignée TMTG3960-1 (promoteur et séquence génomique codante du facteur IX). Ces ARN sont séparés par électrophorèse sur gel 1% agarose-formaldéhyde, transférés sur membrane nylon et hybrides à une sonde humaine radioactive marquée à l'isotope ³²P (900 pb de la séquende 3' non traduite du gène du facteur IX clonées dans ppolyIII-I*). La figure 5 montre que les ARN messagers du facteur IX humain (environ 3 kb) sont détectés uniquement dans les tissus hépatiques des souris transgéniques de la lignée TMTG3960-1 alors qu'aucun signal radioactif n'est détecté pour les ARN isolés des tissus autres que le foie ni dans le foie d'un animal contrôle. Des souris transgéniques qui expriment le facteur IX humain spécifiquement au niveau du foie ont donc été obtenues.

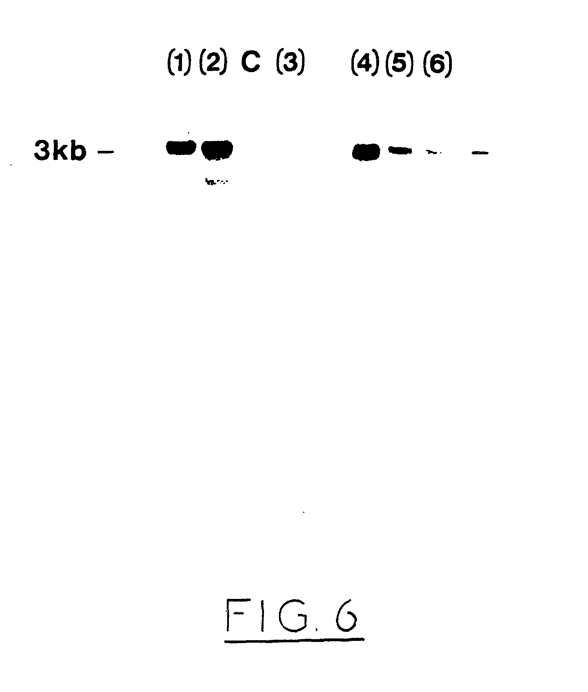

D'autre part, lorsque les ARN messagers issus du foie de toutes les lignées de souris transgéniques sont analysés avec la même sonde, l'intensité du signal est globalement comparable au niveau d'expression (Figure 6).

### C. Analyse du facteur IX humain par Western blot

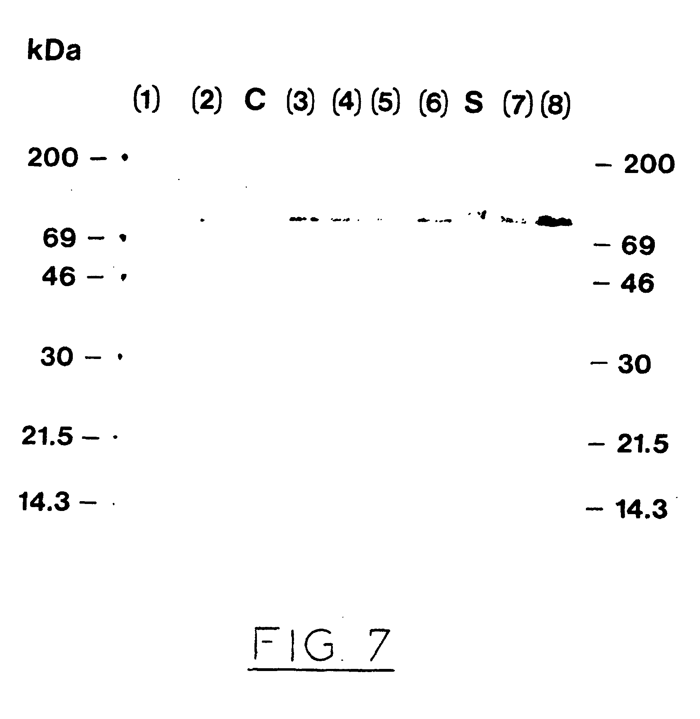

Du plasma est prélevé chez des souris transgéniques issues des lignées TMTG3960-1, TMTG3954-9 et TMTG3954-58. Une fraction est précipitée par le barium et, après redissolution, analysée par Western blot. La figure 7 montre que le facteur IX humain issu d'une souris transgénique a une masse moléculaire similaire à celle du facteur IX du plasma humain, ce qui indique que le produit obtenu est glycosylé (un facteur IX peu ou non glycosylé aurait une masse moléculaire plus faible).

### D. Activité coagulante du facteur IX humain présent dans le plasma des souris transgéniques

On détermine, par test ELISA, la concentration du facteur IX humain dans le plasma des souris des différentes lignées. Les résultats sont indiqués dans le tableau 2. Les différentes souris sont identifiées par le numéro de la lignée (par exemple TMTG3960-1) suivi de la génération (par exemple I pour génération F1), suivi d'un numéro particulier. On peut noter que la concentration de facteur IX humain quantifiée chez les souris de première génération de la lignée TMTG3960-1 est supérieure à celle déterminée chez la souris fondatrice (voir tableau 1) car cette dernière est mosaïque. Sur ces mêmes prélévements, l'activité du facteur IX humain est déterminée par un test de coagulation par la technique de dosage un temps de Austen and Rhymes, telle que précédenment rapportée. L'activité est déterminée en U/ml par rapport à un échantillon de plasma humain utilisé comme témoin (Stago 1U/ml). Le plasma murin présente un bruit de fond important (de l'ordre de 1U/ml). La préincubation des échantillons de plasma des souris transgéniques avec un anticorps polyclonal spécifique anti-facteur IX humain neutralise celui-ci et réduit le niveau d'activité à celui du facteur IX murin. La différence entre la valeur obtenue en absence d'anticorps et celle obtenue en présence d'anticorps correspond à l'activité due exclusivement au facteur IX humain. Cette activité est rapportée en µg/ml (1U/ml = S µg/ml de facteur IX dans du plasma humain normal pris en référence). Les résultats sont présentés dans le Tableau 2 ci-dessous.

**Tableau 2**

| Souris | Concentration en facteur IX (µg/ml) | Activité facteur IX (µg/ml) | % |
|---|---|---|---|
| TMTG3960-1 I 3 | 25,5 | 22 | 86 |
| TMTG3960-1 I 29 | 22,5 | 21 | 93 |
| TMTG3960-1 I 46 | 29,2 | 22 | 75 |
| TMTG3962-18 II 9 | 2 | 2,3 | 116 |
| TMTG3962-18 II 8 | 3,9 | 4,4 | 113 |
| TMTG3954-58 I 24 | 9,6 | 7,3 | 76 |
| TMTG3954-9 II 16 | 19,8 | 19,8 | 100 |

Le Tableau 2 montre que, dans le plasma de souris transgéniques, le facteur IX humain est biologiquement actif puisque l'activité coagulante (mesurée en µg/ml) équivaut à la quantité de facteur IX mesurée par test ELISA, et ceci pour toutes les lignées.

### E. Test de précipitation au citrate de barium

La qualité du facteur IX humain présent dans le plasma d'une souris transgénique est également vérifiée par test de précipitation au sel de barium [Bajaj S.P. et al., J. Biol. Chem. (1981) 256: 253]. Les protéines γ-carboxylées s'adsorbent et coprécipitent avec le citrate de barium (sel insoluble) alors que les protéines qui ne le sont pas restent en solution. La quantité de facteur IX humain (tot.) dans le plasma des souris transgéniques est déterminée avant traitement. Après traitement du plasma par le citrate de barium, la fraction adsorbée (ads), donc γ-carboxylée, est redissoute puis quantifiée par test ELISA. Les quantités sont rapportées au volume de l'échantillon et donc données en ng. Le rapport Ads/tot exprimé en pourcentage (%) quantifie la proportion de facteur IX humain γ-carboxylé.

**Tableau 3**

| Souris | Quantité totale de facteur IX | Quantité de facteur IX adsorbé | Rapport Ads/tot en % |
|---|---|---|---|
| TMTG3960-1 I 3 | 1429 | 981 | 69 |
| TMTG3960-1 I 46 | 1630 | 1104 | 67 |
| TMTG3960-1 III 6 | 573 | 504 | 88 |
| TMTG3962-18 II 9 | 152 | 152 | 100 |
| TMTG3962-18 Il 8 | 103 | 102 | 99 |
| TMTG3954-58 I 24 | 370 | 343 | 93 |
| TMTG3954-9 II 16 | 356 | 440 | 82 |

Le tableau 3 montre que la majorité du facteur IX humain présent dans le plasma murin est γ-carboxylé puisqu'il est détecté dans la fraction insoluble. Par comparaison avec le facteur IX présent dans le plasma humain, on obtient un taux de précipitation de 80 à 100 % selon les expériences.

### F. Purification et caractérisation du facteur IX obtenu à partir des lignées TMTG3960-1 et TMTG3954-9

Le facteur IX humain obtenu à partir de souris issues des lignées TMTG3960-1 et TMTG3954-9 est purifié par chromatographie d'immunoaffinité. La fraction éluée est passée sur HPLC phase réverse pour désaler le produit et la séquence N-terminale déterminée par dégradation d'Edman. Les résultats obtenus dans chaque cas indiquent que le facteur IX humain présent dans le sang des souris transgéniques correspond bien à la forme mature. On ne détecte aucune trace du précurseur du facteur IX humain. Le facteur IX humain des souris transgéniques est donc homogène et correctement processé.

### EXEMPLE 3: Lignées cellulaires trans-immortalisées exprimant le facteur IX humain

### A. Souris simple-transgéniques exprimant un oncogène

La lignée de souris transgénique TMTG2984 exprimant l'oncogène c-myc est générée en utilisant le fragment NotI-NotI du plasmide pTG2984 décrit dans la demande de brevet EP-A 0 298 807.

La lignée de souris transgénique TMTG4912 exprimant les antigènes de tumeur T et t du SV40 est générée en utilisant le fragment SaII-EcoRI du plasmide pTG4912 décrit ci-dessous.

Le plasmide pTG4912 dérive du plasmide pTG171 décrit dans le brevet EU 0140762 et du plasmide pML2. Le plasmide pML2 dérive d'un plasmide "pJYM like", décrit dans l'article de Lusky M. et al. [Nature 293, (1981) pp. 79-81] dans lequel la région poison de pBR322 qui est délétée est un fragment d'ADN compris entre les nucléotides 1086 et 2457. L'insert BamHI de SV40 est de plus délété, et on obtient ainsi le plasmide pML2.

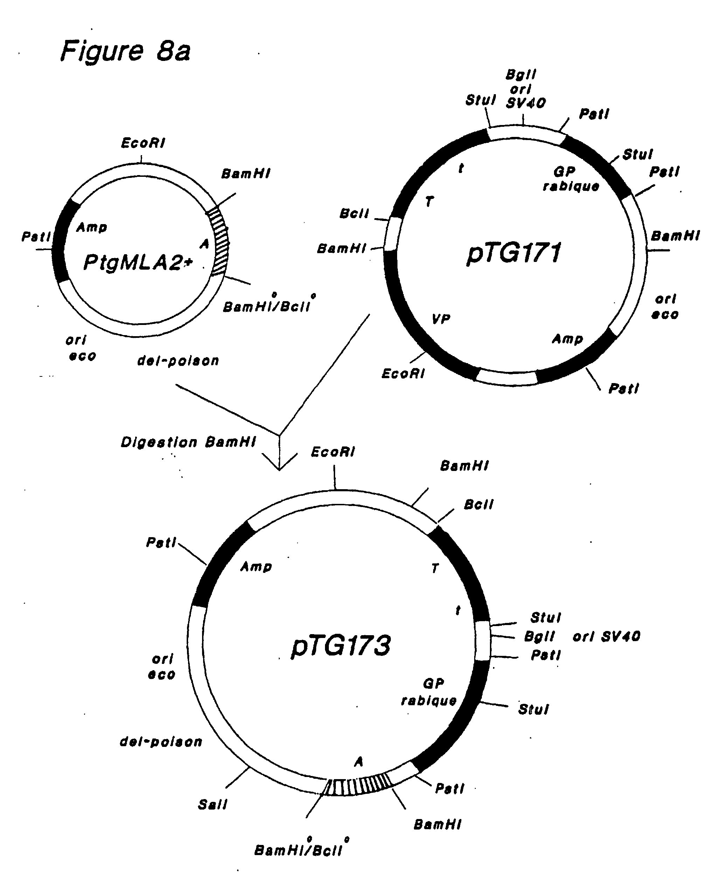

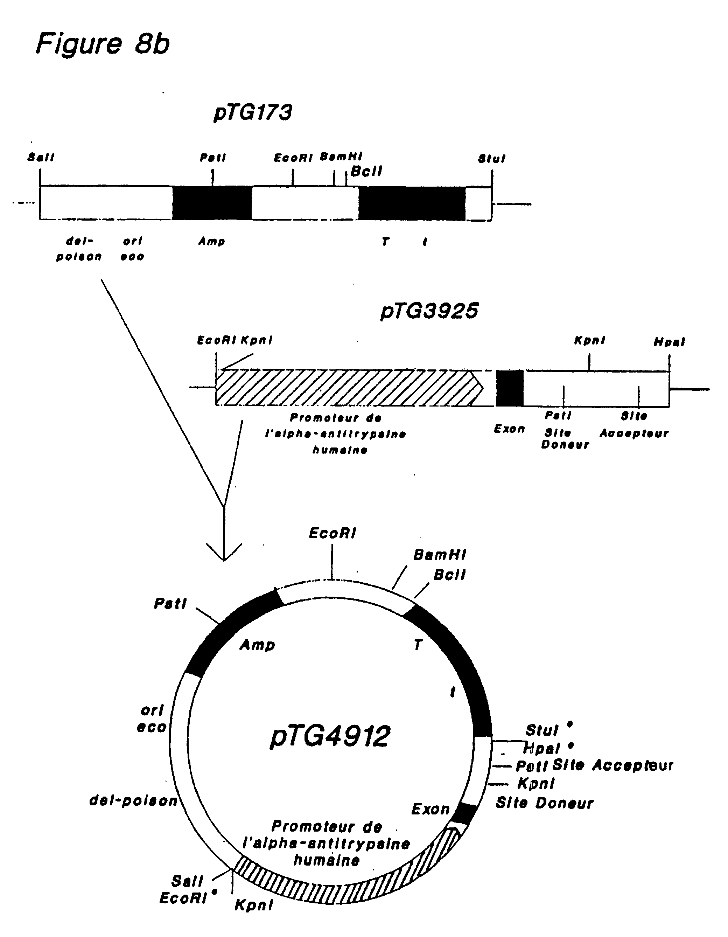

Le fragment 2553-2770 BamHI-BclI du génome de SV40 [Buchman et al. Tooze J. Eds., DNA Tumor Viruses - Second Edition Revised, pp.799-841, (1981) est inséré dans le vecteur pML2 préalablement digéré par BamHI, pour donner le vecteur pTGML2A⁺ représenté à la Figure 8.

Le fragment BamHI-BamHI du plasmide pTG171, portant les gènes codant pour les antigènes T et t et pour la glycoprotéine de la rage est inséré dans le vecteur pTGML2A⁺ préalablement digéré par BamHI pour générer le plasmide pTG173 représenté à la Figure 8.

Le plasmide pTG173 est digéré par les enzymes StuI, qui fait une coupure franche, et Sali, qui laisse des extrémités cohésives 5' sortant. Les petits fragments StuI-StuI et StuI-SaII sont éliminés. Le grand fragment SalI-StuI comportant les régions codant pour les antigènes T et t, le gène de la résistance à l'ampiciline et l'origine de réplication est isolé, et soumis à l'action de la polymérase Klenow (Amersham) en présence des 4 nucléotides triposphate ATP, GTP, CTP et TTP. La polymérase rempli le site Sall avec les nucléotides et on obtient ainsi un fragment d'ADN ayant deux extrémités franches.

Le plasmide pTG3925 précédemment décrit est digéré par EcoRI, qui laisse des extrémités cohésives 5' sonant, et Hpal, qui fait une coupure franche. Le fragment EcoRI-HpaI comportant le promoteur, le premier exon non codant, une partie du premier intron portant le site donneur d'épissage du gène de l'α-antitrypsine et un site synthétique accepteur d'épissage est isolé, et soumis à l'action de la nucléase "Mung Bean" (Biolabs) qui coupe les extrémités cohésives du site EcoRI. On obtient ainsi un fragment ayant deux extrémités franches. Ce fragment est inséré dans le plasmide pTG173 digéré par StuI et Sall comme préparé ci-dessus, pour obtenir le plasmide pTG4912 représenté à la Figure 8.

### B. Souris pluri-transgéniques exprimant le facteur IX humain et au moins un oncogène

Des souris double transgéniques sont générées par croisement des souris de la lignée TMTG3960-1 avec des souris de la lignée TMTG2984. L'etat de double transgénique est vérifié par analyse Dot blot avec des sondes appropriées. Les souris fondatrices de la lignée TMTG3960-1 X TMTG2984 expriment à la fois l'α-antitrypsine humaine et le facteur IX humain. Ces deux caractères d'expression sont transmis à la descendance de façon mendelienne. Trois de ces souris doublement transgéniques développent des tumeurs hépatiques à l'âge de 12 mois.

Afin d'obtenir des souris qui développent des tumeurs à un âge plus précoce, un mâle de la lignée TMTG3960-1 X TMTG2984 est croisé avec des femelles de la lignée TMTG4912. Un des triples transgéniques qui sont générés par ce croisement exprime 1mg/ml d'α-antitrypsine humaine et 40.3µg/ml de facteur IX humain. Celui-ci est sacrifié à l'âge de 6 semaines et son foie (foie n° 1) qui présente déjà une hyperplasie, est prélevé. Un autre triple transgénique qui exprime 3mg/ml d'α-antitrypsine humaine et 60µg/ml de facteur IX humain subit le même sort à l'âge de trois mois (foie n° 2).

### C. Sélection de lignées cellulaires trans-immortalisées

Chacun des foies est traité comme suit:

Le tissu hépatique tumoral est dissocié par traitement à la collagénase tel que décrit par Howard & Pesh, J. Bio. Chem. (1968) 105: 3105, à l'exception de l'étape de perfusion. Puis les cellules sont établies dans du milieu Williams'E (Gibco) supplémenté avec de l'insuline 10ng/ml, de l'hydrocortisone 300ng/ml de la transferrine 10µg/ml, du facteur de croissance epidermal (EGF) 25ng/ml, de la glutamine 2mM, de la streptomycine 50 U/ml, de la pénicilline 250 U/ml et 5% de sérum de veau fétal (FCS) dialysé. L'addition de ces différents constituants serait essentielle pour la croissance et le maintien d'une morphologie d'hépatocyte. Les flacons de culture Primaria (Falcon) sont utilisés pour cette première culture. La culture est poursuivie à 37°C, en atmosphère humidifiée chargée à 10% de CO₂.

Au cours de cette première culture, les cellules sont cultivées en amas. Puis, lorsqu'elles sont à 80% de confluence, elles sont détachées en présence de trypsine à 0.05% et repiquées plusieurs fois par passages successifs dans le milieu complémenté ci-dessus décrit. Lors des premiers repiquages, les cellules sont transférées extemporanément (environ 10min) dans des flacons plastiques sur les parois desquels les macrophages s'attachent immédiatement et peuvent être ainsi éliminés. Après 5 passages, le FCS est remplacé par de la sérumalbumine bovine (BSA) 1mg/ml. Après 5 passages suplémentaires des cultures pures d'hépatocytes sont obtenues: TMhep39 et TMhep48, respectivement sélectionnées à partir des foies n° 1 et 2.

La quantité de facteur IX produite par ces lignées cellulaires est mesurée directement dans le surnageant des cultures par test ELISA. Pour TMhep39, la productivité est d'environ 0.15µg/10⁶ cellules/24hrs; et pour TMhep48, elle est comprise entre 0.29 et 0.55µg/10⁶ cellules/24hrs. Lorsque les cultures sont à haute densité cellulaire, la production atteint respectivement 0.3-0.4µg/ml/24hrs et environ 0.7µg/ml/24hrs.

L'activité "facteur IX" du facteur IX produit par les lignées cellulaires TMhep39 etTMhep48 est mesurée par le test de Austen & Rhymes précédenment décrit. Dans les deux cas, elle est proche de 100%.

Le facteur IX présent dans le surnageant des cultures des deux lignées cellulaires est purifié sur une colonne d'immunoaffinité. Le séquençage de l'extrémité N-terminale confirme que le facteur IX ainsi produit correspond bien à la forme mature. Un Western blotting confirme de même que sa mobilité électrophorétique est identique à celle du facteur IX dérivé du plasma humain.

### D. Caractérisation des lignées cellulaires TMhep39 et TMhep48

La production d'albumine qui est une protéine spécifiquement sécrétée par le foie est détectée dans le milieu de culture par un test ELISA en utilisant comme premier anticorps un antisérum de lapin anti-albumine de souris et comme deuxième anticorps un anticorps de lapin anti-albumine de souris marqué à la péroxydase. Pour les 2 lignées cellulaires, le niveau d'albumine sécrétée varie 3 et 11 µg/ml/24h.

Dans les cellules de la lignée TMhep39, la présence d'ARN messagers spécifiques des cellules du foie est détectée par la technique du "spot blot" décrite par Costanzi et Gillespie (1987) [in : Berger S.L., Kimmel A.R. eds. Guide to Molecular Cloning Techniques, 152. Orlando : Academic press, 582-587.], à partir de l'ARN total préparé par la methode à l'hydrochloride de guanidium telle que décrite par Chirgwin et al., Biochemistry (1979) 18: 701-710. La recherche des ARNm codant pour la transferrine de souris (1), l'α-antitrypsine de souris (2), l'albumine de souris (3), la β-actine de souris (4), l'α-antitrypsine humaine (5), la vimentine de hamster (6), facteur IX humain (7), c-myc de souris (8), antigène T de SV40 (9), glutathion-S-transférase (10), α-fétoprotéine de souris (11) est initiée à l'aide de sondes dont les séquences sont respectivement les suivantes :

AGTGGTCTCTGCTGACTCACAC 1
CTGTTTAGCAGGAACTTAGAGATGAGCTCC 2
TAGTGGGGTTGATAGGAAAGGT 3
GATGTCACGCACGATTTCCCTCTCA 4
GGTGATGATATCATGGGTGAGTTCATTTTC 5
TGCCGGTTCGAGGTGCCGGGG 6
CTCAGTACAGGAGCAAACCACCTTG 7
AGATATCCTCACTGGGCGCGGGCGG 8
AATAGCAGACACTCTATGCCTGTGTGGAGT 9
CTAAAGAGCGACCCAGGTGCCT 10
GGTGAGTTCTTGAGTATTCA 11

La sonde correspondant à la β-actine, protéine ubiquitaire, est utilisée comme contrôle positif. L'absence d'hybridation est correctement observée lorsque l'on utilise la sonde 6 (contrôle négatif) et la sonde 11 (correspondant à une protéine uniquement exprimée au niveau foetal c'est à dire indifférencié). Une réponse positive est observée pour l'albumine, les α-antitrypsines humaine et de souris, la glutathion transférase, le facteur IX humain, le c-myc de souris, l'antigène T du SV40 et la transferrine.

Les résultats reportés ci-dessus indiquent clairement que les lignées cellulaires obtenues après repiquages successifs conservent un phénotype stable et différencié.

La souche E. coli BJ transformée par le plasmide pTG3960 a été déposée le 19 juillet 1989 auprès de la C.N.C.M. *25,* rue du Dr. ROUX 75724 PARIS sous le numéro I-893.

La lignée cellulaire TMhep48 a été déposée le 9 Août 1990 auprès de la C.N.C.M. 25, rue du Dr. ROUX 75724 PARIS sous le numéro I989.

## Revendications

1. Une lignée cellulaire trans-immortalisée d'origine hépatique, capable d'exprimer un facteur IX humain dont l'activité est similaire à celle du facteur IX présent dans le plasma sanguin humain, qui a intégré dans son génome un fragment d'ADN exogène comprenant une région de type génomique ou minigène codant pour un facteur IX humain, ladite région étant placée sous le contrôle d'une région promotrice spécifique du foie, et un second fragment d'ADN exogène comprenant une région codant pour un oncogène, ladite région codante étant placée sous le contrôle d'une région promotrice appropriée.

2. Une lignée cellulaire selon la revendication 1, dont la région codant pour le facteur IX humain est de type minigène.

3. Une lignée cellulaire selon la revendication 1, dont la région codant pour le facteur IX humain est de type génomique.

4. Une lignée cellulaire selon n'importe laquelle des revendications 1 à 3, qui a intégré dans son génome un fragment d'ADN exogène comprenant une région de type génomique ou minigène codant pour le facteur IX humain, ladite région codante étant placée sous le contrôle d'une région promotrice sélectionnée parmi le promoteur du gène du facteur IX, le promoteur du gène de l'α-antitrypsine et les parties de ceux-ci suffisantes pour induire l'expression de ladite région codante.

5. Une lignée cellulaire selon n'importe laquelle des revendications 1 à 4, qui a intégré dans son génome un fragment d'ADN exogène comprenant une région de type génomique ou minigène codant pour le facteur IX humain, ladite région codante étant placée sous le contrôle du promoteur du gène du facteur IX ou une partie de celui-ci suffisante pour induire l'expression de ladite région codante.

6. Une lignée cellulaire selon l'une des revendications 1 à 5, **caractérisée en ce que** le second fragment d'ADN comprend une région codant pour un oncogène choisi dans le groupe constitué par le gène du virus SV 40 codant pour l'antigène T ou le gène C-myc de souris placée sous le contrôle d'une région promotrice spécifique du foie.

7. Une lignée cellulaire selon la revendication 6, **caractérisée en ce que** la région promotrice spécifique du foie est le promoteur du gène de 1'α-1 antitrypsine.

8. Une lignée cellulaire selon la revendication 3, sélectionnée parmi TMhep48 déposée le 9 Août 1990 auprès de la CNCM sous le numéro I-989 et TMhep39 issue de souris triplement transgéniques selon le procédé suivant : Des souris double transgéniques sont générées par croisement des souris de la lignée TMTG3960-1, générée à partir du plasmide pTG3960 déposé le 19 Juillet 1989 sous le numéro I-893 auprès de la CNCM, avec des souris de la lignée TMTG2984 générée à partir du plasmide pTG2984, puis des souris de la lignée TMTG3960-1xTMTG2984 sont croisées avec des souris de la lignée TMTG4912 générée à partir du plasmide pTG4912 représentée à la figure 8 b, puis on prélève le foie tumoral desdites souris triplement tarnsgéniques ou d'un animal dans lequel les cellules dudit foie tumoral ont été propagées et on sélectionne par une mise en culture ladite lignée TMhep39.

9. Un animal transgénique non humain dont le plasma sanguin contient un facteur IX humain dont l'activité est similaire à celle du facteur IX présent dans le plasma sanguin humain, qui a intégré dans son génome un premier fragment d'ADN exogène comprenant une région de type génomique ou minigène codant pour un facteur IX humain, ladite région codante étant placée sous le contrôle d'une région promotrice spécifique du foie, et un second fragment d'ADN exogène comprenant une région codant pour un oncogène, ladite région codante étant placée sous le contrôle d'une région promotrice appropriée.

10. Un animal transgénique non humain selon la revendication 9, dont la région codant pour le facteur IX humain est de type minigène.

11. Un animal transgénique non humain selon la revendication 9, dont la région codant pour le facteur IX humain; est de type génomique.

12. Un animal transgénique non humain selon n'importe laquelle des revendications 9 à 11, qui a intégré dans son génome un fragment d'ADN exogène comprenant une région de type génomique ou minigène codant pour le facteur IX humain; la dite région codante étant placée sous le contrôle d'une région promotrice sélectionnée parmi le promoteur du gène du facteur IX, le promoteur du gène de l'α-antitrypsine et les parties de ceux-ci suffisante pour induire l'expression de la dite région codante.

13. Un animal transgénique non humain selon n'importe laquelle des revendications 9 à 12, qui a intégré dans son génome un fragment d'ADN exogène comprenant une région de type génomique on minigène codant pour le facteur IX humain; la dite région codante étant placée sous le contrôle du promoteur du gène du facteur IX ou partie de celui-ci suffisantes pour induire l'expression de la dite région codante.

14. Un procédé de préparation d'une lignée cellulaire selon n'importe laquelle des revendications 1 à 8, dans lequel
- on prélève le foie tumoral d'un animal transgénique selon la revendication 12 ou le foie tumoral d'un animal dans lequel des cellules hépatiques tumorales dérivées d'un animal transgénique selon la revendication 9 ont été propagées et,
- on sélectionne, par mise en culture des cellules du dit foie, la dite lignée cellulaire.

15. Un procédé de préparation d'un facteur IX d'origine humaine ayant une activité similaire à celle du facteur IX présent dans le plasma humain, dans lequel on cultive une lignée cellulaire selon n'importe laquelle des revendications 1 à 8 et on récolte, par la suite, le dit facteur IX dans le milieu de culture.

## Patentansprüche

1. Trans-immortalisierte Zellinie hepatischen Ursprungs, die fähig ist, einen humanen Faktor IX zu exprimieren, dessen Aktivität ähnlich derjenigen des Faktors IX ist, der im menschlichen Blutplasma anwesend ist, welche in ihrem Genom ein Fragment exogener DNS integriert aufweist, das eine Region vom genomischen oder vom Minigen-Typ, welche für einen menschlichen Faktor IX kodiert, wobei die Region unter die Kontrolle einer für die Leber spezifischen Promotor-Region gestellt ist, und ein zweites Fragment exogener DNS umfaßt, das eine Region umfaßt, die für ein Onkogen kodiert, wobei die kodierende Region unter die Kontrolle einer geeigneten Promotor-Region gestellt ist.

2. Zellinie nach Anspruch 1, deren Region, die für den menschlichen Faktor IX kodiert, vom Minigen-Typ ist.

3. Zellinie nach Anspruch 1, deren Region, die für den menschlichen Faktor IX kodiert, vom genomischen Typ ist.

4. Zellinie nach irgendeinem der Ansprüche 1 bis 3, die in ihrem Genom ein Fragment exogener DNS integriert aufweist, das eine Region vom genomischen oder vom Minigen-Typ umfaßt, welche für den menschlichen Faktor IX kodiert, wobei die kodierende Region unter die Kontrolle einer Promotor-Region gestellt ist, die ausgewählt ist aus dem Promotor des Gens des Faktors IX, dem Promotor des α-Antitrypsin-Gens und Teilen derselben, die ausreichen, um die Expression der kodierenden Region zu induzieren.

5. Zellinie nach irgendeinem der Ansprüche 1 bis 4, die in ihrem Genom ein Fragment exogener DNS integriert aufweist, das eine Region vom genomischen oder vom Minigen-Typ umfaßt, die für den menschlichen Faktor IX kodiert, wobei die kodierende Region unter die Kontrolle des Promotors des Gens des Faktors IX oder eines Teils desselben, welcher ausreicht, um die Expression der kodierenden Region zu induzieren, gestellt ist.

6. Zellinie nach einem der Ansprüche 1 bis 5, **dadurch gekennzeichnet, daß** das zweite DNA-Fragment eine Region umfaßt, die für ein Onkogen kodiert, welches aus der Gruppe ausgewählt ist, die aus dem Gen des Virus SV40, das für das T-Antigen kodiert, oder dem Gen C-myc der Maus besteht, und unter die Kontrolle einer für die Leber spezifischen Promotor-Region gestellt ist.

7. Zellinie nach Anspruch 6, **dadurch gekennzeichnet, daß** die für die Leber spezifische Promotor-Region der Promotor des Gens von α-1-Antitrypsin ist.

8. Zellinie nach Anspruch 3, ausgewählt aus TMhep48, hinterlegt am 9. August 1990 bei der CNCM unter der Nummer I-989, und TMhep39, die gemäß dem folgenden Verfahren aus dreifach transgenen Mäusen hervorgeht: doppelt transgene Mäuse werden erzeugt durch Kreuzen von Mäusen der Linie TMTG3960-1, erzeugt ausgehend von dem Plasmid pTG3960, hinterlegt am 19. Juli 1989 unter der Nummer I-893 bei der CNCM, mit Mäusen der Linie TMTG2984, erzeugt ausgehend von dem Plasmid pTG2984, dann werden Mäuse der Linie TMTG3960-1xTMTG2984 mit Mäusen der Linie TMTG4912, erzeugt ausgehend vom Plasmid pTG4912, das in der Fig. 8b dargestellt ist, gekreuzt, dann entnimmt man die Tumorleber der dreifach transgenen Mäuse oder eines Tiers, in dem die Zellen der Tumorleber vermehrt worden sind, und man selektioniert mittels Kultivieren der Linie TMhep39.

9. Nicht-menschliches transgenes Tier, dessen Blutplasma einen menschlichen Faktor IX enthält, dessen Aktivität ähnlich ist derjenigen des Faktors IX, der in menschlichem Blutplasma vorhanden ist, welches in seinem Genom ein erstes Fragment exogener DNS, das eine Region vom genomischen oder vom Minigen-Typ umfaßt, die für einen menschlichen Faktor IX kodiert, wobei die kodierende Region unter die Kontrolle einer für die Leber spezifischen Promotor-Region gestellt ist, und ein zweites Fragment exogener DNS integriert aufweist, das eine Region umfaßt, die für ein Onkogen kodiert, wobei die kodierende Region unter die Kontrolle einer geeigneten Promotor-Region gestellt ist.

10. Nicht-menschliches transgenes Tier nach Anspruch 9, dessen Region, die für den menschlichen Faktor IX kodiert, vom Minigen-Typ ist.

11. Nicht-menschliches transgenes Tier nach Anspruch 9, dessen Region, die für den menschlichen Faktor IX kodiert, vom genomischen Typ ist.

12. Nicht-menschliches transgenes Tier nach irgendeinem der Ansprüche 9 bis 11, das in seinem Genom ein Fragment exogener DNS integriert aufweist, welches eine Region vom genomischen oder vom Minigen-Typ umfaßt, die für den menschlichen Faktor IX kodiert; wobei die kodierende Region unter die Kontrolle einer Promotor-Region gestellt ist, die ausgewählt ist aus dem Promotor des Gens des Faktors IX, dem Promotor des α-Antitrypsin-Gens und Teilen derselben, die ausreichen, um die Expression der kodierenden Region zu induzieren.

13. Nicht-menschliches transgenes Tier nach irgendeinem der Ansprüche 9 bis 12, das in seinem Genom ein Fragment exogener DNS integriert aufweist, das eine Region vom genomischen oder vom Minigen-Typ umfaßt, welche für den menschlichen Faktor IX kodiert; wobei die kodierende Region unter die Kontrolle des Promotors des Gens des Faktors IX oder eines Teils desselben, welcher ausreicht, um die Expression der kodierenden Region zu induzieren, gestellt ist.

14. Verfahren zur Herstellung einer Zellinie nach irgendeinem der Ansprüche 1 bis 8, in dem
- man die Tumorleber eines transgenen Tiers nach Anspruch 12 oder die Tumorleber eines Tiers, in dem die hepatischen Tumorzellen, die von einem transgenen Tier nach Anspruch 9 abstammen, vermehrt worden sind, entnimmt und
- man durch Kultivieren der Zellen der besagten Leber die Zellinie selektioniert.

15. Verfahren zur Herstellung eines Faktors IX menschlichen Ursprungs, der eine Aktivität aufweist, die derjenigen des Faktors IX ähnlich ist, *der* im menschlichen Plasma vorhanden ist, in welchem man eine Zellinie nach irgendeinem der Ansprüche 1 bis 8 kultiviert und anschließend den Faktor IX aus dem Kulturmedium gewinnt.

## Claims

1. A trans-immortalized cell line of hepatic origin, capable of expressing a factor IX of human origin whose activity is similar to that of factor IX present in human blood plasma, which has integrated in its genome an exogenous DNA fragment comprising a region of the genomic or minigene type coding for a factor IX of human origin; the said coding region being placed under the control of a promoter region specific to the liver, and a second exogenous DNA fragment comprising a region coding for an oncogene, the said coding region being placed under the control of an appropriate promoter region.

2. A cell line according to Claim 1, whose region coding for human factor IX is of the minigene type.

3. A cell line according to Claim 1, whose region coding for human factor IX is of the genomic type.

4. A cell line according to any one of Claims 1 to 3, which has integrated in its genome an exogenous DNA fragment comprising a region of the genomic or minigene type coding for human factor IX; the said coding region being placed under the control of a promoter region selected from the factor IX gene promoter, the α-antitrypsin gene promoter and the portions of these promoters sufficient for inducing expression of the said coding region.

5. A cell line according to any one of Claims 1 to 4, which has integrated in its genome an exogenous DNA fragment comprising a region of the genomic or minigene type coding for human factor IX; the said coding region being placed under the control of the factor IX gene promoter or a portion of the latter sufficient for inducing expression of the said coding region.

6. A cell line according to one of Claims 1 to 5, **characterized in that** the second DNA fragment comprises a region coding for an oncogene chosen from the group consisting of the SV 40 virus gene coding for the T antigen or the C-myc gene of mice placed under the control of a promoter region specific to the liver.

7. A cell line according to Claim 6, **characterized in that** the promoter region specific to the liver is the α-1-antitrypsin gene promoter.

8. A cell line according to Claim 3, selected from TMhep48 deposited on 9 August 1990 at the CNCM under the number I-989 and TMhep39 derived from triply transgenic mice according to the following method: doubly transgenic mice are generated by crossing mice of the TMTG3960-1 line, generated from the plasmid pTG3960 deposited on 19 July 1989 under the number I-893 at the CNCM, with mice of the TMTG2984 line generated from the plasmid pTG2984, and then mice of the TMTG3960-1×TMTG2984 line are crossed with mice of the TMTG4912 line generated from the plasmid pTG4912 represented in Figure 8b, and then the tumorous liver is removed from the said triply transgenic mice or from an animal in which the cells of the said tumorous liver have been propagated and the said TMhep39 line is selected by placing in culture.

9. A non-human transgenic animal whose blood plasma contains a human factor IX whose activity is similar to that of factor IX present in human blood plasma, which has integrated in its genome a first exogenous DNA fragment comprising a region of the genomic or minigene type coding for a human factor IX, the said coding region being placed under the control of a promoter region specific to the liver, and a second exogenous DNA fragment comprising a region coding for an oncogene, the said coding region being placed under the control of an appropriate promoter region.

10. A non-human transgenic animal according to Claim 9, whose region coding for human factor IX is of the minigene type.

11. A non-human transgenic animal according to Claim 9, in which the region coding for human factor IX is of the genomic type.

12. A non-human transgenic animal according to any one of Claims 9 to 11, which has integrated in its genome an exogenous DNA fragment comprising a region of the genomic or minigene type coding for human factor IX; the said coding region being placed under the control of a promoter selected from the factor IX gene promoter, the α-antitrypsin gene promoter and the portions of these promoters sufficient for inducing expression of the said coding region.

13. A non-human transgenic animal according to any one of Claims 9 to 12, which has integrated in its genome an exogenous DNA fragment comprising a region of the genomic or minigene type coding for human factor IX; the said coding region being placed under the control of the factor IX gene promoter or part of the latter sufficient for inducing the expression of the said coding region.

14. A method for preparing a cell line according to any one of Claims 1 to 8, in which
- the tumorous liver of a transgenic animal according to Claim 12, or the tumorous liver of an animal in which tumorous liver cells derived from a transgenic animal according to Claim 9 have been propagated, is removed, and
- the said cell line is selected by culturing the cells of the said liver.

15. A method for preparing a factor IX of human origin having an activity similar to that of factor IX present in human plasma, in which a cell line according to any one of Claims 1 to 8 is cultured and the said factor IX in the culture medium is subsequently harvested.
